# EUROPEAN PATENT SPECIFICATION

(11) **EP 2 180 854 B1**
(45) Date of publication and mention of the grant of the patent: **04.05.2016**
(21) Application number: 08776611.9
(22) Date of filing: 15.07.2008
(51) Int. Cl.: A61F 5/01

(54) **CLUBFOOT ORTHOTICS**
KLUMPFUSS-ORTHESE
ORTHÉTIQUE DU PIED RAMPIN

(30) Priority: 24.07.2007 IL 18481207
(43) Date of publication of application: 05.05.2010
(73) Proprietor: Daizade, Izak, 46701 Herzelia Pituach (IL)
(72) Inventor: Daizade, Izak, 46701 Herzelia Pituach (IL)
(74) Representative: Virdee-Crofts, Kulwinder Kaur
(86) International application number: PCT/IL2008/000978
(87) International publication number: WO 2009/013730

(56) References cited:
- EP-A- 0 130 915
- US-A- 3 308 829
- US-A- 4 922 895
- US-A- 4 981 132
- US-A- 5 277 699

## Description

### FIELD AND BACKGROUND OF THE INVENTION

The present invention relates to an apparatus for the correction of congenital clubfoot.

Congenital idiopathic talipes equinovarus, herein clubfoot deformity, is a complex deformity that occurs in otherwise generally healthy infants.
Figure 1 shows the bones of a normal right foot 100 from a dorsal view. In a rear foot 110, a calcaneal axis 124 passing through a calcaneus 112 is substantially aligned with a first ray axis 104 passing through a forefoot 120.
Figure 2 shows a right clubfoot 200 from an aerial, herein dorsal, view of the foot. Forefoot 120 has moved medially 132, in a position herein referred to as a forefoot adductus (numeral 130 denotes the lateral side of the foot).
Figure 3 shows right clubfoot 200 from a side view, demonstrating a calcaneus 112 up in the air while forefoot 120 is on a supporting surface herein a rearfoot equinus 134. Further, rearfoot 110 is turned in so that calcaneus 112 has moved under talus 114, herein referred to as a calcaneal varus. Additionally, forefoot 120 has turned so that first ray axis 104 is up in the air, herein referred to as a forefoot supinatus 136.

The above-noted clubfoot deformities, equinus of the rearfoot, varus of the calcaneus, adductus of the forefoot, and forefoot supinatus, must be treated in the infant.

If left untreated, clubfoot deformity will result in secondary bony changes wherein the adult walks on the lateral aspect, instead of the plantar aspect, of the foot.

In 1950, Ignacio Ponseti, MD, at the University of Iowa, developed a method of treating clubfoot using strait-last shoes positioned in abduction and connected to a transverse bar.

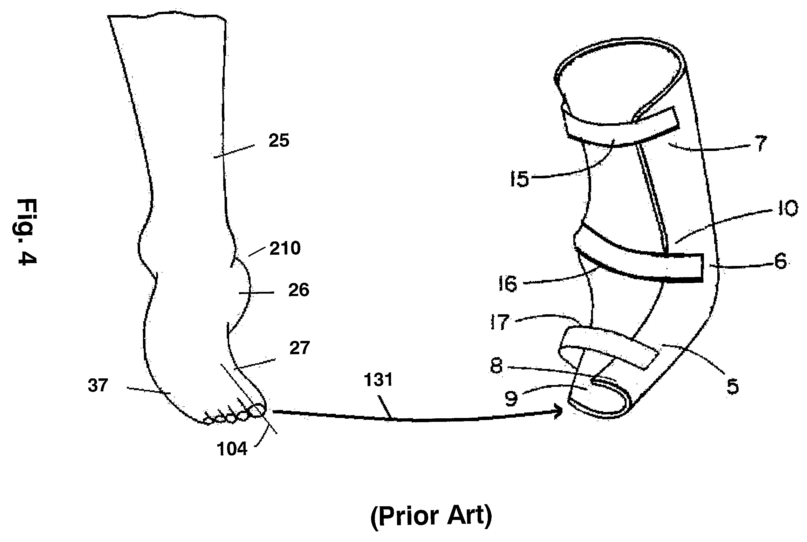

Additionally, U.S. Patent 4,922,895 (Chong), , teaches a stand-alone brace 10, shown in Figure 4, for correction of a foot 210 having an adductus deformity 27 of a forefoot 37.

Brace 10 includes a leg strap 15 that presses a leg 25 in a direction 131 into a leg support 7; a heel strap 16 that pushes a heel 26 into a heel support 6; and a forefoot strap 17 that pushes forefoot 37 into a forefoot support 9, with a first ray 104 under a first ray flange 8.

EP0130915 discloses an anti-adductus transverse module for the correction of the metatarsus adductus having an external medial counterbracing which is movable transversally with respect to the sole and forms an envelope of the medial upper portion of the foot and means for immobilizing the counterbracing with respect to the sole in the supporting position.

### SUMMARY OF THE INVENTION

According to the present invention there is provided a clubfoot orthotic as defined in claim 1. Preferred embodiments of the invention are defined in the dependent claims.

### BRIEF DESCRIPTION OF THE DRAWINGS

The invention of a clubfoot orthotic configured to substantially correct clubfoot deformities is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

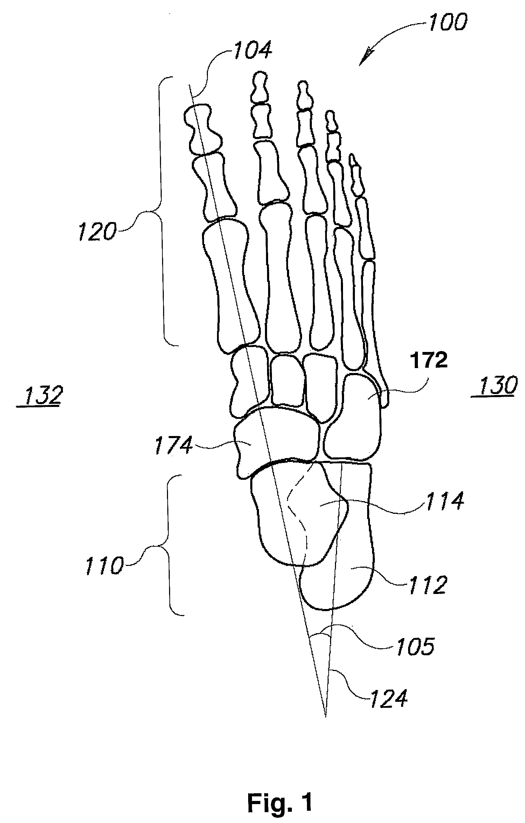

Figure 1 shows a dorsal view of the bones of a normal right foot;

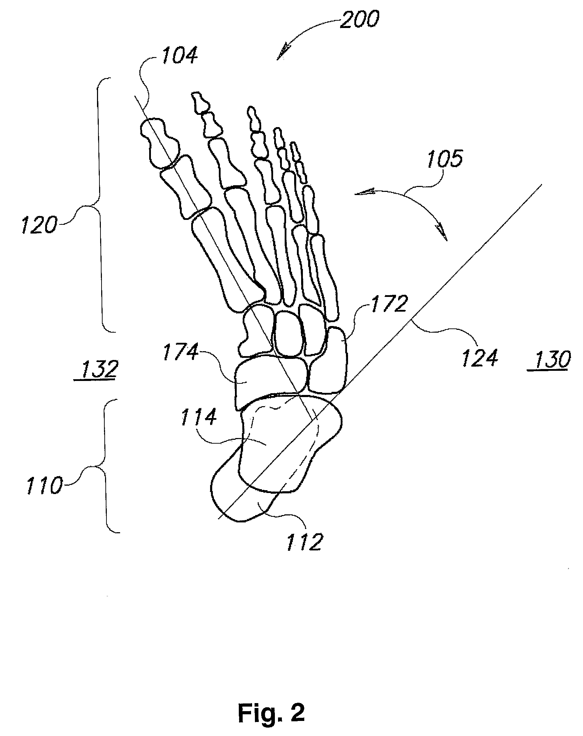

Figure 2 shows a dorsal view of the bones of a right clubfoot;

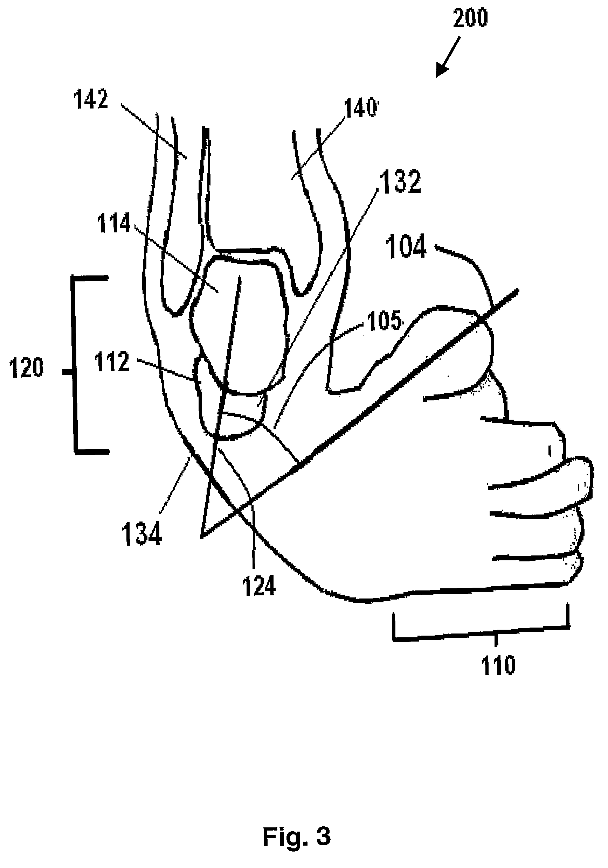

Figure 3 shows a side view of the bones of a right clubfoot;
Figure 4 shows a right foot exhibiting forefoot adductus and a known brace used for correcting forefoot adductus;

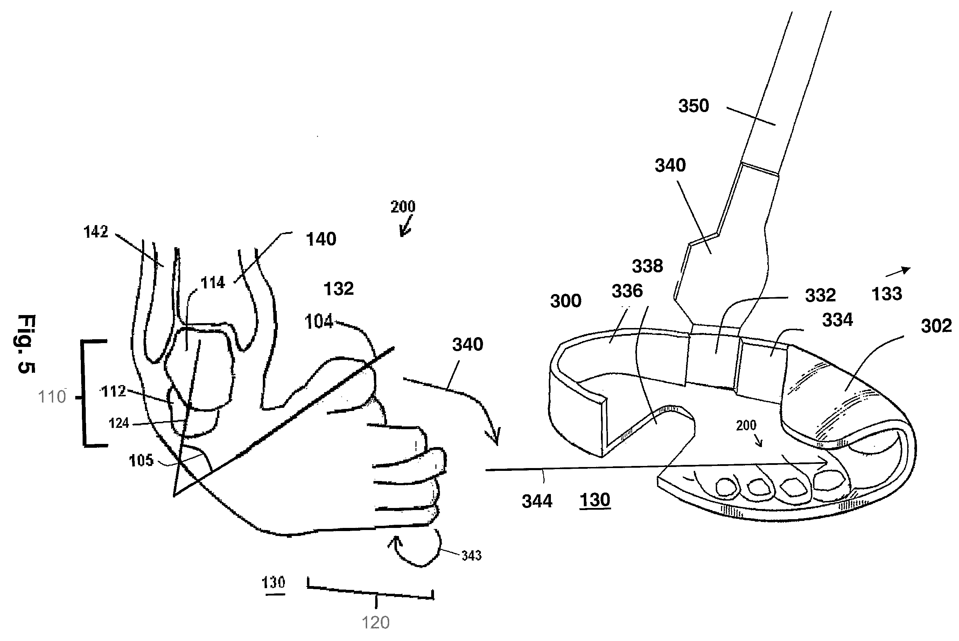

Figure 5 shows a right clubfoot being set in a clubfoot orthotic, according to embodiments of the invention;

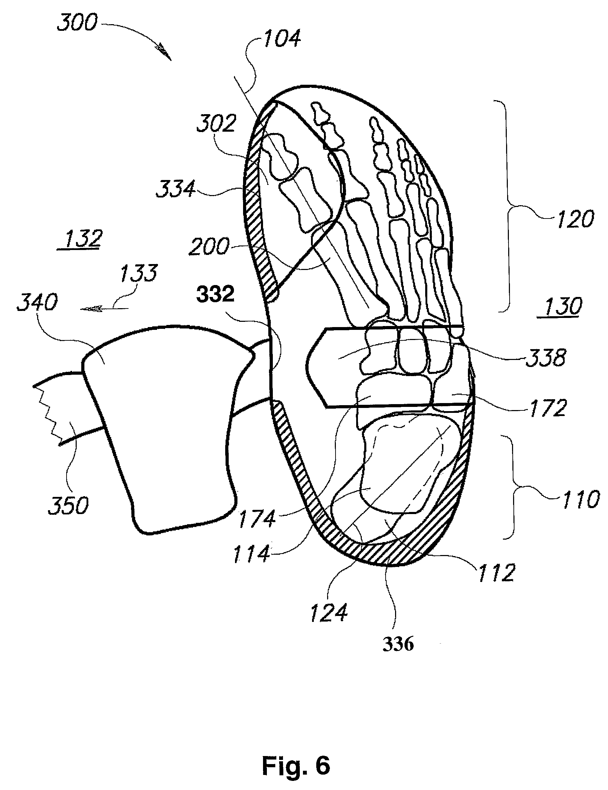

Figure 6 shows a dorsal view of the bones of the right clubfoot and clubfoot orthotic of Figure 5, according to embodiments of the invention;

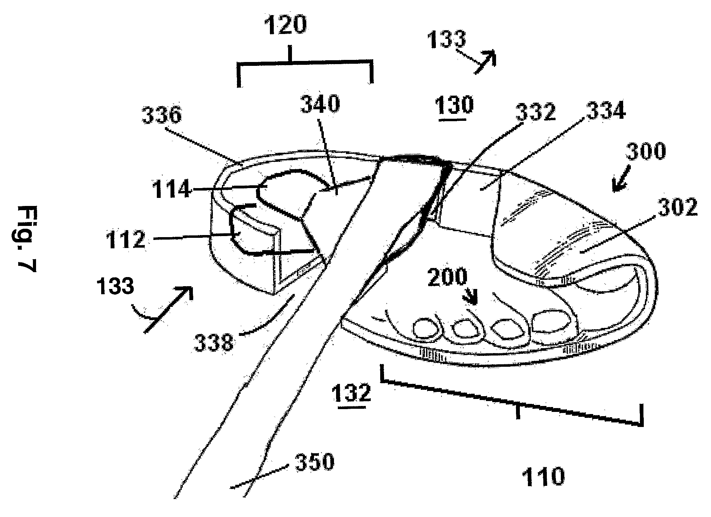

Figures 7-10 show a right clubfoot being strapped into the clubfoot orthotic of

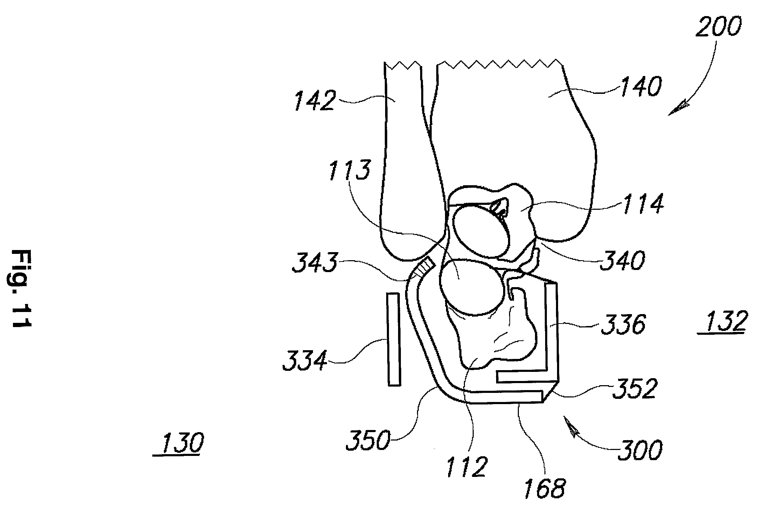

Figures 5 and 6, according to embodiments of the invention; Figure 11 shows a midfoot cutaway view from the anterior of a clubfoot secured in a partial schematic view of the clubfoot orthotic shown in Figure 5, according to embodiments of the invention;

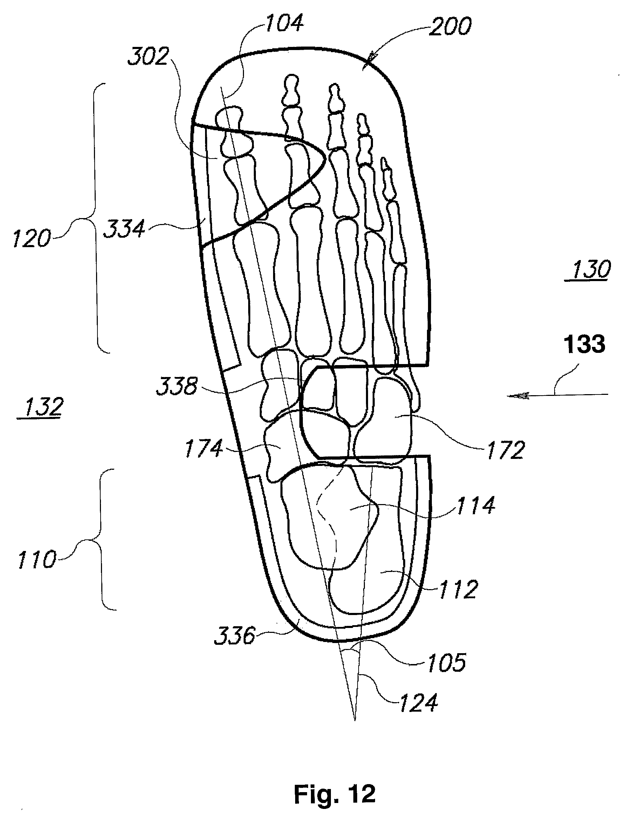

Figure 12 shows a dorsal view of the bones of the right clubfoot secured in a partial view of the clubfoot orthotic of Figure 5, according to embodiments of the invention; and

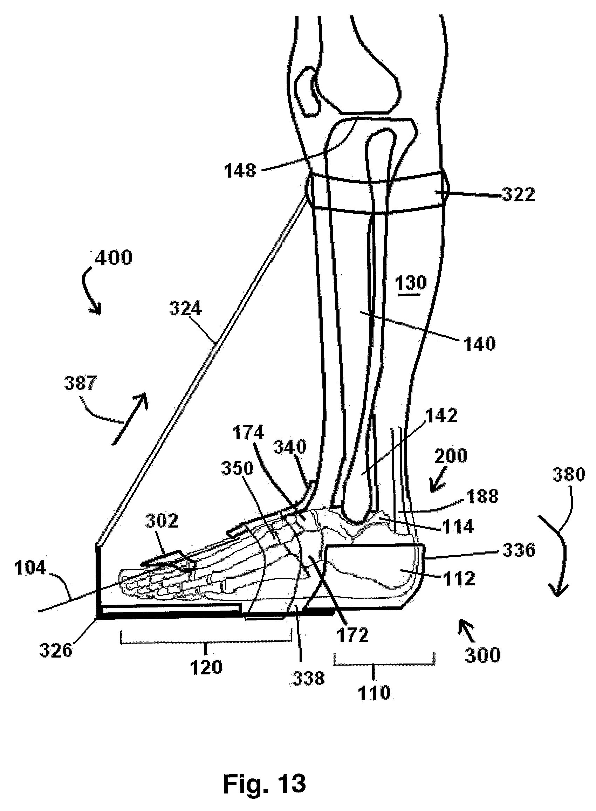

Figure 13 shows a side view of the clubfoot and clubfoot orthotic of Figure 5, including an equinus corrector, according to embodiments of the invention.

### DESCRIPTION OF THE PREFERRED EMBODIMENTS

Embodiments of the present invention are a clubfoot orthotic that can be used to substantially correct clubfoot deformities.

The principles and operation of the clubfoot orthotic according to the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. Further, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

### Referring now to the drawings:

Figure 5 shows right clubfoot 200 being set in a clubfoot orthotic 300, according to embodiments of the invention. The ankle joint includes talus 114 that is stabilized between a tibia 140 and a fibula 142. Below talus 114 is calcaneus 112 that is in a varus position.

An abnormally large angle 105 is formed by first ray 104 and calcaneal axis 124 that is initially addressed to begin restoring proper foot architecture.

Initially, right clubfoot 200 is massaged and forefoot 120 circumducted while first ray 104 is rotated in a plantigrade direction 343 to bring forefoot 120 to a plantigrade position. First ray is then moved in medial direction 133 and clubfoot 200 is manipulated so that first ray 104 sets beneath a first ray flange 302 of clubfoot orthotic 300.

As shown in Figure 6 in a dorsal view of the bones of clubfoot 200, the supinatus of first ray 104 has come to a plantigrade position over and calcaneus 112 is sitting in a padded heel cup 336. Calcaneus 112, however, remains in varus and forefoot 120 still maintains a certain amount of adductus. away from clubfoot orthotic in a medial direction 133.

To correct the varus position of calcaneus 112, a posterior cuboid strap 350 will be pulled anterior to rearfoot 110 over and plantarly to a posterior portion of cuboid 172 and a navicular bone 174 into a lateral midfoot cutout 338. Posterior portion of cuboid 172 will be moved in medial direction 133 and the anterior portion of calcaneus 112 will follow and become be pressed toward a medial midtarsal depression 332. Medial midtarsal depression 332 is formed by an unpadded section of clubfoot orthotic 300 between a first ray padding 334 and padded heel cup 336. Pressing calcaneus 112 and posterior portion of cuboid 172 into medial midtarsal depression causes the anterior portion of calcaneus 112 to rotate out of calcaneal varus.

As shown in Figure 7, posterior cuboid strap 350 has been pulled across clubfoot anterior to rearfoot 110 plantarly into a cuboid sulcus so the anterior portion of calcaneus 112 is pressed into medial midtarsal depression 332 in medial direction 133.

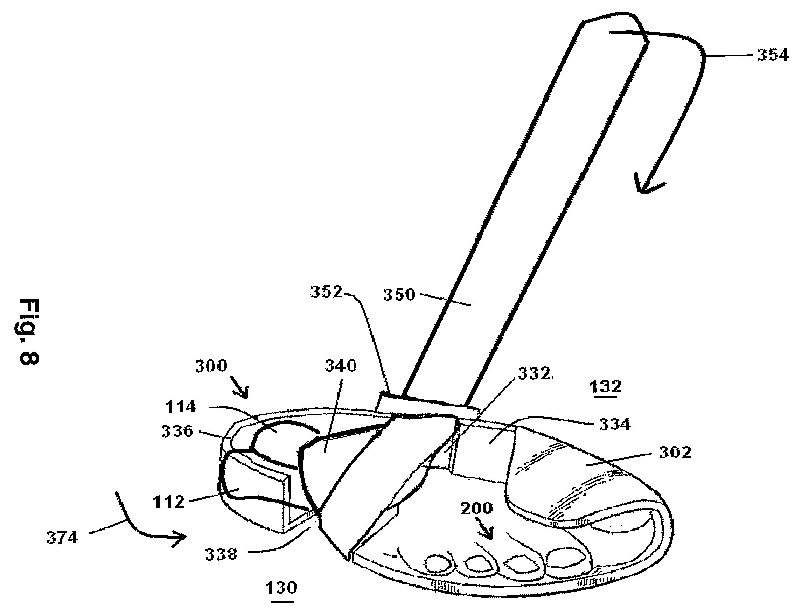

As seen in Figure 8, posterior cuboid strap 350 has been passed through a clasp
352 attached to medial aspect 132 of clubfoot orthotic 300. Posterior cuboid strap 350 is then pulled taut in a plantigrade direction 354 so that a talar pad 340 presses plantigrade rotational direction 374 on talus 114, causing calcaneus 112 to stabilize within padded heel cup 336 in.

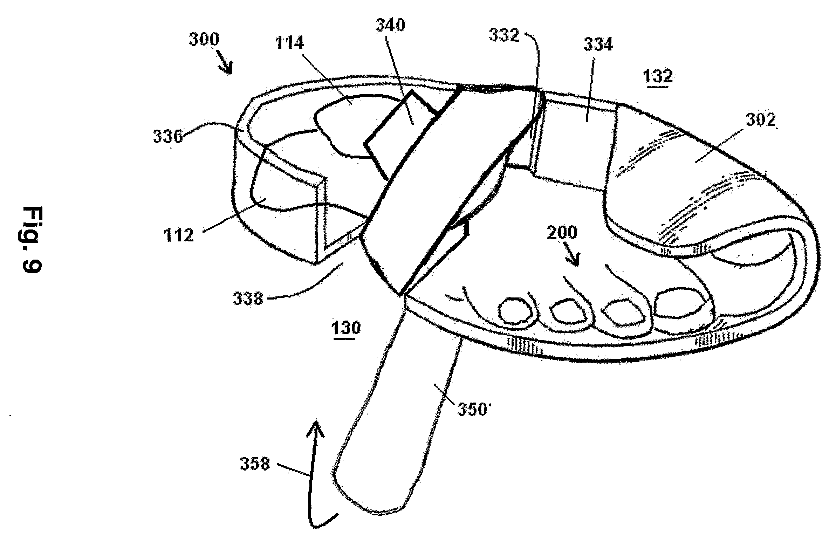

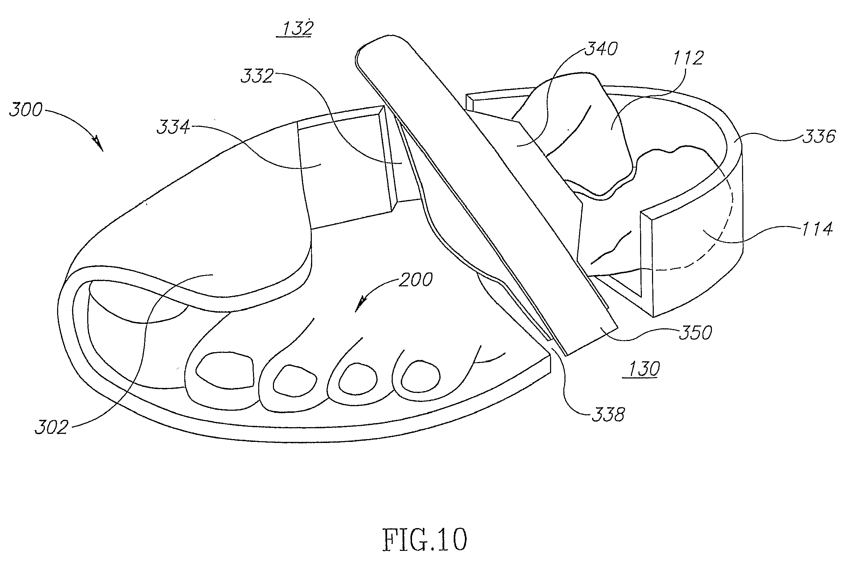

Figure 9 shows posterior cuboid strap 350 brought across the plantar aspect of clubfoot orthotic 300 and rotated in a dorsal direction 358. As seen in Figure 10, posterior cuboid strap 350 has been folded over dorsally over talar pad 340 in a direction 358.

Figure 11 shows a schematic head-on view of clubfoot orthotic 300 cutaway of clubfoot 200 in which posterior cuboid strap 350 is shown beginning at medial aspect of padded heel cup 336 and pressing a calcaneus head 113 toward a plantar aspect 168 of clubfoot orthotic 300. Posterior cuboid strap 350 continues below plantar aspect 168, through clasp 352 and doubles back dorsally where posterior cuboid strap 350 secures with Velcro. Alternatively, posterior cuboid strap 350 is optionally secured in place with snaps or clasps, not shown; the many options for securing posterior cuboid strap 350 being well known to those familiar with the art. The head- on view of calcaneus 112 and talus' 114 demonstrate that clubfoot orthotic 300 has corrected varus of calcaneus 112 in the coronal plane.

Figure 12 shows a dorsal projection of the bones of clubfoot 200 demonstrating reduction of the angle between first ray axis 104 and talar axis 124. Additionally, clubfoot orthotic 300 has created pressure in medial direction 133 to correct adductus of forefoot 120 and supinatus of first ray 104. Correction of the above-noted components of clubfoot 200 are usually maintained without clubfoot orthotic 300 following between at least about two weeks and four weeks; and attention can now be directed to the final clubfoot deformity, equines of rearfoot 110.

Figure 13 shows a side view of clubfoot 200 and clubfoot orthotic 300, including an equinus corrector 400 comprising an orthotic connector 326 connected to the plantar aspect of the anterior of clubfoot orthotic 300. A stretchable band 324 extending from orthotic connector 326 has been pulled in a substantially dorsal direction 387 and connected to a padded knee strap 322 that surrounds tibia 140 and fibula 142 below a knee joint 148. Stretchable band 324 pulls forefoot 120 dorsally, thereby gradually stretching an Achilles tendon 188, and/or a tibial posterior tendon (not shown) so that calcaneus 112 rotates in a plantigrade direction 380.

Stretchable band 324 is optionally connected lateral to the medial border of forefoot 120 in order to impart a valgus component to calcaneus 112 and reverse varus of calcaneus 112. In embodiments, stretchable band 324 is optionally connected near or along the lateral border of forefoot 120 to create an optimal corrective force on calcaneus 112.

Stretchable band 324 optionally comprises a stretchable rubber material and is optionally adjusted to apply tension to Achilles tendon 188 without causing discomfort to the infant.

Equinus corrector 400 is optionally used in conjunction with clubfoot orthotic 300 each night until Achilles tendon 188 maintains a stretched configuration without use of equinus corrector 400.

In embodiments Achilles tendon 188 maintains a stretched configuration following between about two and eights weeks use of equinus corrector 400. In embodiments Achilles tendon 188 maintains a stretched configuration following between about four and six weeks equinus corrector 400.

In the event of residual tightness of Achilles tendon 188, a surgical percutaneous Achilles tenotomy is optionally performed, thereby releasing the tightness. Optionally clubfoot orthotic 300 and equinus corrector 400 are used following the Achilles tenotomy in order to maintain the proper length of Achilles tendon 188.

After correction of Achilles tendon 188 clubfoot orthotic 300 and equinus corrector 400 are then removed from corrected clubfoot 200.

The authors have found that some embodiments of the present invention may have advantages over U.S. Pat. No. 4,922,895 (Chong), (Figure 4), noted above, who substantially addresses only the forefoot adductus component of clubfoot.

The authors have additionally found that some embodiments of the present invention may have advantages over clubfoot treatments advocated by of Ignacio Ponseti, MD, noted above, in which the discomfortable strait-last shoes and transverse bar may result in non-compliance. Non-compliance is associated with a clubfoot relapse rate of 78%.

In distinct contrast to the Ponseti apparatus and method, the inventor has found that embodiments of the present clubfoot orthotic invention have not resulted in non-compliance; with the orthotic being substantially well tolerated by the infants shown in the experimental examples below.

It is expected that during the life of this patent many relevant materials and designs for clubfoot orthotic 300 will be developed and the scope of the term "clubfoot orthotic" is intended to include all such new technologies a priori.

As used herein the term "about" refers to ± 10 %.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non-limiting fashion.

Below are two photographs from a first child who, in the upper photograph presents with bilateral clubfoot showing forefoot adductus, first ray supinatus, calcaneal varus, and equinus. In the lower photograph, the first child has been fit bilaterally with clubfoot orthotics. Following about six weeks treatment after taking these photographs, the feet showed substantial correction of multiple clubfoot deformities. 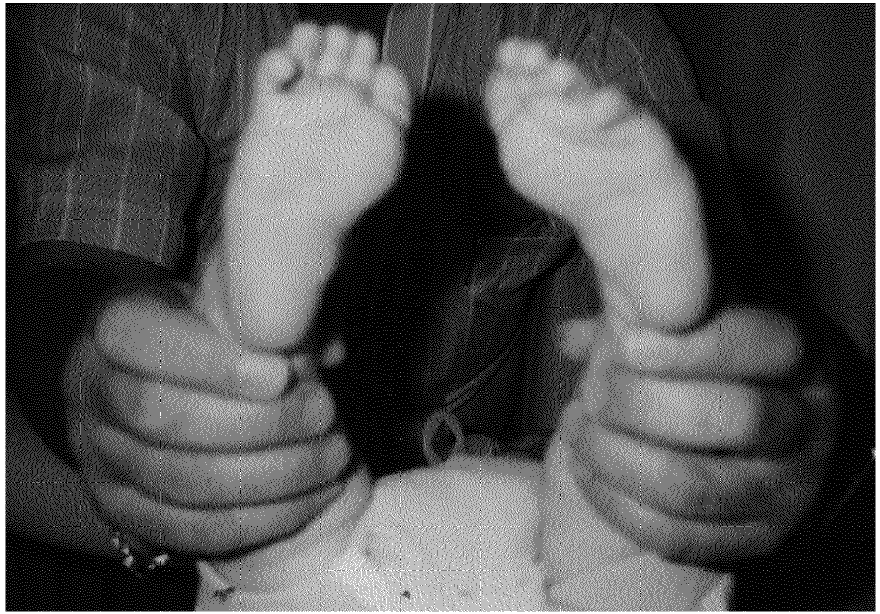 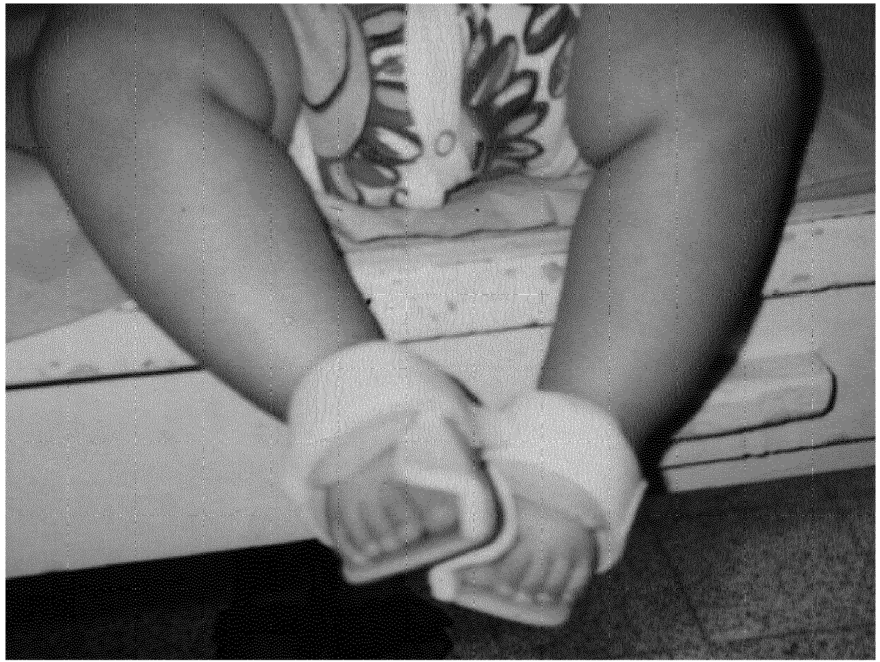 Below are two photographs from a second child. The upper photograph shows a bilateral clubfoot in which the left foot shows a significant splay between the first ray and the lateral four metatarsals.

In the bottom photograph, the child is shown in clubfoot orthotic embodiments of the present invention. Again, following about six weeks treatment after taking these photographs, the feet showed substantial correction of multiple clubfoot deformities. 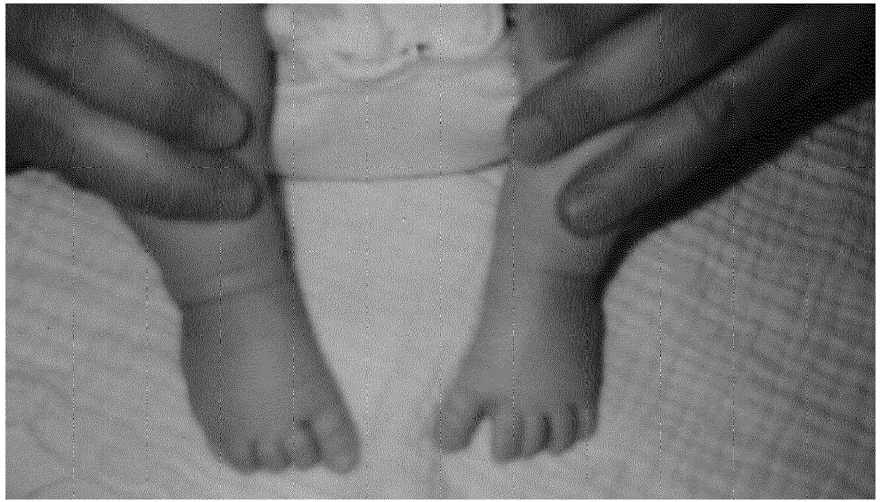 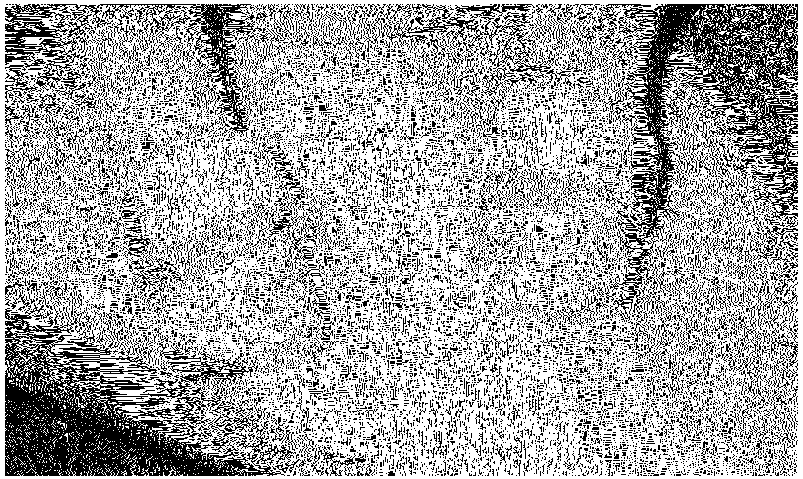 Although the invention has been described in conjunction with specific embodiments thereof, it is evident that many alternatives, modifications and variations will be apparent to those skilled in the art. Accordingly, it is intended to embrace all such alternatives, modifications, and variations that fall within the scope of the appended claims. In addition, citation or identification of any reference in this application shall not be construed as an admission that such reference is available as prior art to the present invention.

## Claims

1. A clubfoot orthotic (300) for treating clubfoot, the clubfoot orthotic (300) comprising-
an orthotic shell having a plantar surface configured to support a portion of a foot disposed in said orthotic, said orthotic shell including a wall configured to surround a medial boundary of the foot and a heel portion of the foot;
a lateral midfoot cutout (338) in said orthotic (300) extending laterally through a portion of a midfoot portion of said plantar surface;
a first ray flange (302) extending from at least a portion of an upper boundary of said wall along a medial boundary of a midfoot, said first ray flange (302) configured, when the foot is disposed in said orthotic, to be operatively associated with at least a portion of a first ray (104) of the foot;
a posterior cuboid strap (350) having one end attached to a midfoot portion of said wall;
**characterised by** a medial midtarsal depression (332) formed by an unpadded section of clubfoot orthotic(300) between a first ray padding (334) and a padded heel cup (336);
wherein pulling said posterior cuboid strap (350) anterior to the rearfoot (110) over and plantarly to a posterior portion of cuboid (172) and navicular bone (174) into said lateral midfoot cutout (338) will cause a posterior portion of said cuboid (172) and the anterior portion of calcaneus (112) to be pressed into said medial midtarsal depression (332).

2. The apparatus according to claim 1, including a talar pad (340) on a plantar surface of a dorsal portion of said strap, said padding configured, when the foot is disposed in said orthotic, to contact at least a portion of at least one of: a rearfoot and a midfoot.

3. The apparatus according to claim 1, wherein a clasp (352) is located on a medial side of said plantar surface.

## Patentansprüche

1. Klumpfußeinlage (300) zum Behandeln von Klumpfuß, wobei die Klumpfußeinlage (300) Folgendes umfasst:
eine Einlagenschale mit einer plantaren Oberfläche, die zum Stützen eines Abschnitts eines in der genannten Einlage befindlichen Fußes konfiguriert ist, wobei die genannte Einlagenschale eine Wand aufweist, die um eine mediale Begrenzung des Fußes und einen Fersenabschnitt des Fußes konfiguriert ist;
einen lateralen Mittelfußausschnitt (338) in der genannten Einlage (300), der lateral durch einen Abschnitt eines Mittelfußabschnitts der genannten plantaren Oberfläche verläuft;
einen ersten Strahlflansch (302), der von wenigstens einem Abschnitt einer oberen Begrenzung der genannten Wand entlang einer medialen Begrenzung eines Mittelfußes verläuft, wobei der genannte erste Strahlflansch (302) so konfiguriert ist, dass er, wenn der Fuß in der genannten Einlage steckt, operativ mit wenigstens einem Abschnitt eines ersten Strahls (104) des Fußes assoziiert ist;
einen posterioren Kuboidriemen (350), von dem ein Ende an einem Mittelfußabschnitt der genannten Wand angebracht ist;
**gekennzeichnet durch** eine mediale Mittelfußvertiefung (332), die von einer ungepolsterten Sektion der Klumpfußeinlage (300) zwischen einer ersten Strahlpolsterung (334) und einer gepolsterten Fersenmulde (336) gebildet wird;
wobei das Ziehen des genannten posterioren Kuboidriemens (350) vor dem Rückfuß (110) über und plantar zu einem posterioren Abschnitt des Kuboid- (172) und Navicular-Knochens (174) in den genannten lateralen Mittelfußausschnitt (338) zur Folge hat, dass ein posteriorer Abschnitt des genannten Kuboids (172) und der anteriore Abschnitt des Fersenbeins (112) in die genannte mediale Mittelfußvertiefung (332) gepresst werden.

2. Vorrichtung nach Anspruch 1, die ein Talus-Polster (340) auf einer plantaren Oberfläche eines dorsalen Abschnitts des genannten Riemens aufweist, wobei die genannte Polsterung so konfiguriert ist, dass sie, wenn sich der Fuß in der genannten Einlage befindet, wenigstens einen Abschnitt eines Rückfußes und/oder eines Mittelfußes kontaktiert.

3. Vorrichtung nach Anspruch 1, wobei sich eine Schnalle (352) auf einer medialen Seite der genannten planaren Oberfläche befindet.

## Revendications

1. Une orthèse pour pied bot (300) destinée à traiter un pied bot, l'orthèse pour pied bot (300) comprenant :
une coque orthopédique ayant une surface plantaire configurée pour soutenir une partie d'un pied disposé dans ladite orthèse, ladite coque orthopédique incluant une paroi configurée pour entourer une périphérie médiane du pied et une partie talon du pied ;
une découpe latérale de la partie centrale du pied (338) dans ladite orthèse (300) s'étendant latéralement à travers une partie d'une partie centrale du pied de ladite surface plantaire ;
une bride du premier rayon (302) s'étendant depuis au moins une partie d'une périphérie supérieure de ladite paroi le long d'une périphérie médiane d'une partie centrale du pied, ladite bride du premier rayon (302) configurée, quand le pied est disposé dans ladite orthèse, pour être associée de manière fonctionnelle avec au moins une partie d'un premier rayon (104) du pied ;
une sangle parallélépipédique postérieure (350) ayant une extrémité attachée à une partie centrale du pied de ladite paroi ;
**caractérisée par** un renfoncement d'une région médio-tarsienne médiane (332) formé par une section non rembourrée de l'orthèse pour pied bot (300) entre un rembourrage du premier rayon (334) et une coque talonnière rembourrée (336) ;
dans laquelle le fait de tirer ladite sangle parallélépipédique postérieure (350) antérieure à la partie arrière du pied (110) au-dessus de, et de manière plantaire, vers une partie postérieure du parallélépipède (172) et l'os naviculaire (174) dans ladite découpe latérale de la partie centrale du pied (338) entraînera qu'une partie postérieure dudit parallélépipède (172) et la partie antérieure du calcanéum (112) seront pressés dans ledit renfoncement d'une région médio-tarsienne médiane (332).

2. L'appareil selon la revendication 1, incluant un coussinet de l'astragale (340) sur une surface plantaire d'une partie dorsale de ladite sangle, ledit rembourrage étant configuré, quand le pied est disposé dans ladite orthèse, pour entrer en contact avec au moins une partie de : soit une partie arrière du pied, soit une partie centrale du pied.

3. L'appareil selon la revendication 1, dans lequel le fermoir (352) est situé sur un côté médian de ladite surface plantaire.
